# FASCICULE DE BREVET EUROPEEN

(11) **EP 2 103 687 B1**
(45) Date de publication et mention de la délivrance du brevet: **13.07.2016**
(21) Numéro de dépôt: 09155394.1
(22) Date de dépôt: 17.03.2009
(51) Int. Cl.: C12N 5/071, G01N 33/50

(54) **Equivalent de peau pigmentée fonctionnelle**
Pigmentiertes funktionales Hautäquivalent
Functional pigmented skin equivalent

(30) Priorité: 29.04.2008 FR 0852886; 17.03.2008 FR 0851705
(43) Date de publication de la demande: 23.09.2009
(73) Titulaire: L'Oréal, 75008 Paris (FR)
(72) Inventeur: Duval, Christine, 92250 La Garenne Colombes (FR); Bernerd, Françoise, 75018 Paris (FR)
(74) Mandataire: Brohmi, Karim

(56) Documents cités:
- EP-A- 1 878 790
- WO-A-2007/061168
- FR-A- 2 879 747
- NAKAZAWA KEIKO ET AL: "Pigmented human skin equivalent: New method of reconstitution by grafting an epithelial sheet onto a non-contractile dermal equivalent" PIGMENT CELL RESEARCH, vol. 10, no. 6, décembre 1997 (1997-12), pages 382-390, XP002480993 ISSN: 0893-5785
- WANG Z ET AL: "Effects of aloesin on melanogenesis in pigmented skin equivalents" INTERNATIONAL JOURNAL OF COSMETIC SCIENCE 200804 GB, vol. 30, no. 2, 13 mars 2008 (2008-03-13), pages 121-130, XP002496633 ISSN: 0142-5463 1468-2494
- HEDLEY SUSAN J ET AL: "Fibroblasts play a regulatory role in the control of pigmentation in reconstructed human skin from skin types I and II" PIGMENT CELL RESEARCH, vol. 15, no. 1, février 2002 (2002-02), pages 49-56, XP002496634 ISSN: 0893-5785
- DUVAL C ET AL: "THE USE OF RECONSTRUCTED HUMAN SKIN TO EVALUATE UV-INDUCED MODIFICATIONS AND SUNSCREEN EFFICACY" EXPERIMENTAL DERMATOLOGY, COPENHAGEN, DK, vol. 12, no. 2, 1 janvier 2003 (2003-01-01), pages 64-70, XP009051378 ISSN: 0906-6705
- ARCHAMBAULT MICHAEL ET AL: "Keratinocytes and fibroblasts in a human skin equivalent model enhance melanocyte survival and melanin synthesis after ultraviolet irradiation" JOURNAL OF INVESTIGATIVE DERMATOLOGY, vol. 104, no. 5, 1995, pages 859-867, XP009105950 ISSN: 0022-202X
- NAKAZAWA K ET AL: "PIGMENTED HUMAN SKIN EQUIVALENT - AS A MODEL OF THE MECHANISMS OF CONTROL OF CELL-CELL AND CELL-MATRIX INTERACTIONS" MEDICAL AND BIOLOGICAL ENGINEERING AND COMPUTING, SPRINGER, HEILDELBERG, DE, vol. 36, no. 6, 1 novembre 1998 (1998-11-01), pages 813-820, XP000784856 ISSN: 0140-0118
- SCOTT G A ET AL: "KERATINOCYTES REGULATE MELANOCYTE NUMBER IN HUMAN FETAL AND NEONATAL SKIN EQUIVALENTS" JOURNAL OF INVESTIGATIVE DERMATOLOGY, vol. 97, no. 5, 1991, pages 776-781, XP009105906 ISSN: 0022-202X
- DUVAL CHRISTINE ET AL: "In vitro organotypic models to study skin pigmentation" PIGMENT CELL & MELANOMA RESEARCH, WILEY INTERSCIENCE, vol. 21, no. 2, 1 avril 2008 (2008-04-01), page 254, XP009106959 ISSN: 1755-1471

## Description

La présente invention concerne des peaux reconstruites présentant une pigmentation constitutive, et leur utilisation dans des procédés d'évaluation des phénomènes de pigmentation cutanée; elle se rapporte ainsi aux procédés d'évaluation d'agents susceptibles de moduler cette pigmentation, qu'elle soit constitutive ou induite. Elle concerne également un procédé de préparation de tels modèles de peau.

La couleur de la peau est principalement due à la présence d'un pigment, la mélanine dans l'épiderme. La mélanine est synthétisée par des cellules dendritiques spécifiques localisées dans la couche basale de l'épiderme, les mélanocytes. La mélanogénèse prend place dans des organelles, les mélanosomes qui, chargés de mélanine, sont transférés aux cellules voisines épidermiques, les kératinocytes, via les dendrites.

Cette pigmentation peut être modulée par des facteurs physiques, tels que les rayonnements U.V., chimiques, tels que des produits dépigmentants ou pro-pigmentants, et/ou au cours de modifications physiologiques ou pathologiques du fonctionnement de la peau.

Il est donc souhaitable de disposer de modèles qui permettent, d'une part, d'effectuer les études nécessaires à la meilleure compréhension du rôle de la peau tant dans le domaine mécanique que dans le domaine physiologique, et d'autre part, qui constituent des tests prédictifs de l'activité d'actifs cosmétiques et/ou pharmaceutiques ou encore des effets secondaires d'ingrédients topiques, ou d'agents pris par voie orale

Pour évaluer une modulation de la pigmentation, des modèles in vitro existent à l'heure actuelle. Il s'agit en particulier des modèles suivants :
- Inhibition *in tubo* de la tyrosinase de champignon ou humaine recombinante (modèle non cellulaire uniquement biochimique) (Virador Analytical Biochem 1999)
- Monoculture de mélanocytes normaux ou issus de mélanome en monocouche (Virador Analytical Biochem 1999, Ni-komatsu JID 2007)
- Co-culture de mélanocytes- kératinocytes humains normaux ou immortalisés en monocouche (Régnier Cell Mol Biol 1999, Yoon Pigment Cell Res 2003)
- Epiderme reconstruit pigmenté sur derme dé-épidermisé mort ou sur filtre inerte de polycarbonate : modèle 3D constitué de mélanocytes et de kératinocytes uniquement

On cherche à mettre au point, depuis de nombreuses années, des modèles de peau reconstruite qui permettent, d'une part, d'effectuer les études nécessaires à la meilleure compréhension du rôle de la peau tant dans le domaine mécanique que dans le domaine physiologique, et d'autre part, qui constituent des tests prédictifs de l'activité d'actifs cosmétiques et/ou pharmaceutiques ou encore des effets secondaires d'ingrédients topiques ou pris par voie orale.
EP 285471 décrit ainsi un procédé de préparation d'un équivalent de peau à partir de culture de kératinocytes provenant de la gaine du follicule pileux; on obtient par ensemencement sur un équivalent de derme, un épiderme différencié dont la structure est proche de celle de l'épiderme humain.
Il a par la suite été proposé divers équivalents de peau, et il a été suggéré d'introduire des mélanocytes dans ces modèles.
Les brevets FR 2 689904 et WO 95/10600 décrivent la préparation d'un équivalent d'épiderme contenant des kératinocytes et des mélanocytes, et son utilisation dans des tests de bronzage. Toutefois, ces équivalents d'épiderme sont obtenus sur un support inerte ne reproduisant pas les interactions physiologiques avec le derme, et ne contenant pas de fibroblastes ni d'environnement matriciel. De plus, les conditions de culture ne permettent pas de conserver les mélanocytes dans un phénotype normal en raison de l'emploi d'un promoteur de tumeur (TPA ou PMA)
Cependant, aucun de ces modèles ne permet d'appréhender la modulation de la pigmentation par les acteurs du derme (fibroblastes, matrice extracellulaire, composants de la jonction dermo-épidermique) car ils ne comprennent pas de derme vivant.

Même quand des modèles organotypiques humains comprenant des fibroblastes vivants sont développés, ils ne permettent pas d'évaluer une modulation de la pigmentation dans un contexte physiologique car ils présentent des défauts de fonctionnalité (absence de pigmentation constitutive ou inductible notamment par l'exposition UV).

Des modèles de peau pigmentée ont été développés sur derme mort dé-epidermisé ou sur éponge (matrice de collagène + GAG) recolonisés par des fibroblastes. Cependant, la localisation des mélanocytes y est souvent imparfaite et ces modèles présentent des défauts de fonctionnalité. En effet, ils ne montrent pas ou peu de pigmentation constitutive et la stimulation de la pigmentation par les UV ou par un agent propigmentant physiologique n'est pas observée dans ces modèles.

Des fibroblastes enchâssés dans une matrice de collagène libre (non tendue) ont été aussi employés pour reconstruite une peau pigmentée. Cependant, ces modèles présentent des inconvénients qui ne permettent pas de les utiliser pour évaluer la modulation de la pigmentation.
Bertaux et al (Br J Dermatol 1988), ont réalisé un modèle de peau pigmentée en insérant une biopsie de peau sur l'équivalent dermique afin que les cellules épidermiques sortent de l'épiderme et prolifèrent sur la surface de la lattice. Parmi les inconvénients d'une telle technique, on peut citer l'absence de contrôle des cellules épidermiques et le fait qu'une seule reconstruction de peau est possible avec une biopsie : aucune expérience n'est donc comparable à l'autre (pas de reproductibilité). De plus comme le modèle développé par Haake et Scott (Scott et Haake, J Invest Dermatol 1991) ce modèle ne montre pas de réelle fonctionnalité : il n'y pas de présence de mélanine ni constitutive ni induite.
Dans le modèle d'Archambault et al (J Invest Dermatol. 1995), une stimulation de mélanogénèse est induite après UVB mais pour des doses induisant une altération épidermique (dose cytotoxique) constituant des conditions rédhibitoires pour pouvoir étudier la pigmentation et sa modulation dans un contexte vitro proche du physiologique. D'autres tentatives plus récentes de reconstruction de peau reconstruite pigmentée ont été réalisées sur un support lattice libre, cependant les modèles obtenus ne correspondent pas à des modèles de peau humaine normale car ils sont créés soit à partir de mélanocytes de souris (Yoshimura J Dermatol Sci 2001), soit les mélanocytes sont cultivés dans des conditions inductrices de transformation tumorale , en présence de TPA (Liu Cell Biol Int 2007). Quant à celui développé par Martinhao Sauto et coll. (Sao Paulo Med J 2006), aucun mélanocyte n'est présent ou n'est montré dans l'épiderme.

Il existe donc un besoin de disposer d'un modèle permettant d'appréhender de manière pertinente la peau et sa dérégulation, reproduisant les interactions physiologiques de la peau humaine.
Il existe aussi un besoin de disposer de procédés d'évaluation de la modulation de la pigmentation de la peau, permettant d'appréhender de manière pertinente l'ensemble des mécanismes intervenant dans la pigmentation de la peau et sa dérégulation, reproduisant les interactions physiologiques de la peau humaine.
En particulier, il existe un besoin d'un équivalent de peau comprenant des mélanocytes vivants fonctionnels présentant une localisation similaire à celle existant dans la peau humaine et un compartiment dermique avec des fibroblastes vivants.
C'est pourquoi la présente invention a pour objet un équivalent de peau in vitro caractérisé en ce qu'il comporte au moins un équivalent d'épiderme qui comprend des kératinocytes formant au moins une couche basale et au moins une couche superficielle et au moins un équivalent de derme comprenant une lattice libre de collagène, et en ce qu'il comprend des mélanocytes synthétisant de la mélanine de façon constitutive et des fibroblastes vivants, tous les mélanocytes étant dans la couche basale et l'équivalent d'épiderme et distribués de façon homogène dans l'équivalent d'épiderme, la densité de répartition desdits mélanocytes étant sensiblement constant dans un plan parallèle à ladite surface.
L'équivalent de peau selon l'invention présente donc une pigmentation constitutive, c'est-à-dire en l'absence de toute stimulation par les rayonnements U.V. ou par des actifs propigmentants. Par pigmentation constitutive, on entend la capacité des mélanocytes à produire de la mélanine à l'état basal non stimulé Cette pigmentation correspond à l'existence de mélanocytes qui, dans des conditions physiologiques, produisent une mélanine mature au sein de mélanosomes; par l'intermédiaire des dendrites du mélanocyte, la mélanine est transférée aux kératinocytes qui assurent une distribution homogène de la pigmentation dans l'équivalent d'épiderme. La présence de grains de mélanine dans les mélanocytes et les kératinocytes avoisinants témoigne de la pigmentation constitutive.
L'invention est plus particulièrement un équivalent de peau humaine.
En particulier l'invention concerne une unité (ou complexe) de pigmentation globale et fonctionnelle incluant mélanocytes, kératinocytes, fibroblastes humains normaux et environnement matriciel; celle-ci comporte au moins un équivalent d'épiderme et au moins un équivalent de derme, et comprend une composante pigmentaire constitutive et fonctionnelle (pigmentation constitutive et inductible dans des conditions physiologiques). La présente invention a également pour objet un procédé d'évaluation de la capacité d'un agent à moduler la pigmentation caractérisé en ce que
(a) on applique ledit agent à un équivalent de peau *in vitro,* ledit équivalent de peau comprenant au moins un équivalent d'épiderme comportant des mélanocytes produisant de la mélanine de façon constitutive et au moins un équivalent de derme comportant des fibroblastes vivants, et
(b) on compare la pigmentation (i) de l'équivalent de peau *in vitro* auquel a été appliqué l'agent à évaluer à celle (ii) d'un équivalent de peau témoin n'ayant pas été soumis à l'agent.
Par "un" agent il faut entendre, tout au long de la présente description, "au moins un", à moins qu'il ne soit spécifié différemment. L'application peut se faire à l'étape a par voie topique ou tout autre mode d'administration.

La modulation de la pigmentation au sens large peut être appréciée de manière qualitative et quantitative, aux fins de comparaison. On peut utiliser toutes méthodes permettant l'analyse :
- de la pigmentation et/ou de la couleur de l'équivalent de peau et de la quantité, nature de la mélanine, de son transfert et dégradation dans les kératinocytes; on peut citer à titre d'exemple, par spectrocolorimétrie (mesure de la luminance et ITA) directe ou indirecte, par spectroscopie sélective (mesure au mexamètre), par Siascopie, par mesure en chromatographie liquide haute performance HPLC (mesure des DHI-mélanine, DHICA-mélanine, pheomélanine) par spectrophotométrie visible après dissolution de la peau par le Soluène ou la soude, par analyse d'image après coloration de la mélanine par Fontana-Masson, par imagerie confocale ou mutliphotonique, par résonance paramagnétique électronique, par microscopie électronique à transmission.
- du nombre, forme et maturité des mélanosomes produits dans les mélanocytes, transférés et/ou dégradés dans les kératinocytes,
- de la quantité, maturité et/ou activité enzymatique des protéines du mélanosome telles que par exemple, pmel-17, Tyrosinase, TRP-1, TRP-2 (DCT), MATP, protéine P MART-1, SCL117A, SLC24A5 (nckx5), OA1,
- du nombre, répartition, morphologie (dendricité) des mélanocytes,
- de la quantité, maturité et/ou activité enzymatique des récepteurs, molécules, facteurs de transcription, au niveau membranaire, cytosolique, nucléaire, qui participent aux voies de la mélanogénèse, de transport dans les dendrites et de transfert des mélanines et des mélanosomes dans les kératinocytes. A titre d'exemples, on peut citer, MC1-R, m-KIT, ETB-R, ETA-R, MITF, USF-1, SOX10, Myosine Va, Rho, Rac, Rab27A, melanophiline,
Les méthodes employées pourront être des méthodes d'évaluation de la quantité et de l'activité des protéines et de l'expression de leur gène codant tels que les techniques de réaction à la Dopa, d'immunohistochimie, de coloration histologique, d'analyse d'images, de biologie moléculaire (PCR), de biochimie (WB, dosage immunoenzymatique ELISA) etc...

Le procédé met plus particulièrement en oeuvre un équivalent de peau humaine, et notamment un équivalent de peau comportant au moins des mélanocytes et/ou des kératinocytes et/ou des fibroblastes issus de peau humaine, avantageusement comprenant au moins des mélanocytes de peau humaine.
En particulier le procédé s'applique ainsi sur une unité (ou complexe) de pigmentation globale et fonctionnelle incluant mélanocytes, kératinocytes, fibroblastes humains normaux et environnement matriciel; celle-ci comporte au moins un équivalent d'épiderme et au moins un équivalent de derme, et comprend une composante pigmentaire constitutive et fonctionnelle (pigmentation constitutive et inductible dans des conditions physiologiques).

En effet, il a été trouvé dans le cadre de l'invention qu'il est possible d'obtenir un modèle répondant aux besoins rappelés plus haut, et qui présente les caractéristiques suivantes :
- Il contient les acteurs cellulaires majeurs impliqués dans la pigmentation à savoir le mélanocyte mais aussi ses principaux partenaires cellulaires : les kératinocytes et les fibroblastes.
- Il reproduit l'organisation tridimensionnelle de la peau afin de respecter et de permettre les contacts et les influences existant naturellement entre partenaires cellulaires, la jonction dermo-épidermique et la matrice extracellulaire.
- Il est fonctionnel c'est-à-dire pigmenté à l'état basal (pigmentation constitutive) et capable d'être stimulé par les UV et/ou par des agents propigmentants, dans des conditions reproduisant les phénomènes physiologiques.

Le modèle selon l'invention contient les 3 types cellulaires majeurs présents dans la peau et impliqués dans la pigmentation, dans une architecture proche de celle de la peau, et permet ainsi de reproduire la régulation du mélanocyte et de la pigmentation par ses partenaires cellulaires et la matrice extracellulaire.
En effet, dans la peau, les cellules pigmentaires sont étroitement liées aux cellules épidermiques et dermiques voisines. De part sa localisation dans la couche basale, à l'interface épiderme-derme, des liens physiques et chimiques existent entre mélanocytes et kératinocytes et aussi entre mélanocytes et fibroblastes. Ces interactions avec ses deux types cellulaires ont une fonction de régulation du mélanocyte et de la pigmentation.

Il est reconnu que par l'intermédiaire de facteurs paracrines relargués par les kératinocytes et par les connexions directes physiques établies par les E-cadhérines, les kératinocytes régulent l'adhésion, la croissance, la survie des mélanocytes, la quantité et la qualité de mélanine produite. Parmi ces facteurs, on peut citer par exemple, l'alpha Melanocyte Stimulating Hormone (MSH), l'Endothéline 1 (ET1), le Stem Cell Factor (SCF), les Prostaglandines E2 et F2α (PGE2, PGF2α), le basic Fibroblast Growth Factor (bFGF) ou le Nerve Growth Factor (NGF).
De même, le rôle joué par les fibroblastes dans la pigmentation est maintenant indiscutable : en sécrétant des facteurs identiques à ceux produits par les kératinocytes, tels que le bFGF, SCF ou l'hepatocyte growth factor (HGF) ou différents tels que dickkopf1 et des protéines de la matrice, les fibroblastes modulent le développement, la croissance, la morphologie et la différenciation des mélanocytes.

Par ailleurs, les composants de la jonction dermo-épidermique les molécules de la matrice extracellulaire dermique (ECM) ont aussi un impact sur le mélanocyte et la pigmentation. En effet, les mélanocytes adhèrent aux molécules de la membrane basale, via des intégrines et récepteurs, en particulier à la laminine et au collagène IV, et la présence des molécules de la membrane basale est nécessaire pour la bonne position basale des mélanocytes. La qualité de la jonction dermo-épidermique (ou JDE), dont les composants et leur localisation résultent de l'interaction entre fibroblastes et keratinocytes influence le type d'ancrage (intégrines) au niveau de la JDE.
La modulation des protéines ECM dérivées de fibroblastes affecte aussi la prolifération, la morphologie et l'activité mélanogénique de mélanocytes normaux. En particulier le collagène I, le collagène IV, la fibronectine et la laminine stimulent l'activité tyrosinase, modulent la dendricité des mélanocytes et leur prolifération.

De plus, il existe des boucles cellulaires de régulation: les kératinocytes peuvent agir directement sur les mélanocytes ou indirectement sur les fibroblastes qui à leur tour vont moduler les mélanocytes : par exemple, la sécrétion de IL-1 α et TNF-α par les kératinocytes peut stimuler la production de HGF et de SCF par les fibroblastes, qui en retour, active les mélanocytes. A l'instar, les fibroblastes peuvent relarguer des cytokines stimulatrices des kératinocytes (le KGF par exemple). Ces derniers à leur tour, produiront des facteurs de modulation de la pigmentation.

On sait aussi que l'exposition au soleil de la peau induit une stimulation de la pigmentation : c'est le bronzage, qui correspond à une activation transitoire des mélanocytes et une augmentation de la synthèse de mélanine.
Cette réponse est largement due à la contribution des facteurs paracrines des cellules partenaires, les kératinocytes et les fibroblastes, dont la production est accrue par les UV.
De manière inattendue, le modèle selon l'invention permet de reproduire ces phénomènes et donc de tester des substances ou des stimuli susceptibles de modifier la pigmentation cutanée.
Le procédé d'évaluation selon l'invention permet de prendre en compte tous ces phénomènes et donc d'évaluer la capacité de substances ou des stimuli susceptibles de moduler la pigmentation cutanée, directement et/ou indirectement.

L'équivalent de peau -ou peau reconstruite- selon l'invention présente un équivalent d'épiderme différencié stratifié, comprenant au moins une couche superficielle et au moins une couche basale. Avantageusement, les kératinocytes reproduisent les caractéristiques d'un épiderme *in vivo,* à savoir un épithélium multicouche stratifié avec une couche basale au contact de l'équivalent de derme et dont les strates supérieures témoignent d'une différenciation, reproduisant de la profondeur vers la périphérie une couche supra-basale (ou couche épineuse), une couche granuleuse (*stratum granulosum*) et une couche cornée (*stratum corneum*) d'un épiderme. Par couche superficielle de l'équivalent d'épiderme on entend au moins une couche correspondant à couche suprabasale, granuleuse ou cornée.
Les kératinocytes utilisés dans l'équivalent d'épiderme peuvent être préparés selon toute méthode connue de l'art antérieur. On peut citer à titre d'exemple la culture à partir d'épiderme dissocié provenant de prélèvement de peau normale ou pathologique.
Préférentiellement, selon l'invention les kératinocytes utilisés sont préparés à partir d'épiderme dissocié provenant de prélèvement de peau humaine normale ou pathologique, en particulier selon la méthode décrite dans Rheinwald et Green , Cell , vol 6 , 331-344 , 1975. Ils peuvent provenir de peau caucasienne, asiatique ou africaine, de différents sites anatomiques (tels que notamment dos, visage, sein, dos des mains, paumes etc..), de zones ayant été exposées ou non au soleil. On peut utiliser des kératinocytes provenant de peaux normales. Selon un mode particulier de mise en oeuvre de l'invention, on peut aussi utiliser des kératinocytes provenant de pathologies cutanées telles que par exemple taches de vieillesse (lentigo actinique), melasma, vitiligo, nevus, xeroderma pigmentosum ou mélanome. Ils peuvent être aussi des kératinocytes génétiquement modifiés surexprimant ou sous-exprimant certains gènes.

Les mélanocytes sont intégrés dans l'épiderme et sont localisés dans la couche basale de l'équivalent d'épiderme selon l'invention. ces mélanocytes sont distribués de façon homogène dans l'équivalent d'épiderme, c'est-à-dire que leur densité de répartition est sensiblement constante dans un plan parallèle à la surface dudit équivalent de derme et sensiblement similaire à celle retrouvée dans la peau humaine. La totalité des mélanocytes se trouve ainsi dans la couche basale de l'équivalent d'épiderme selon l'invention, l'absence de mélanocytes dans les couches suprabasales et le stratum corneum de l'équivalent d'épiderme témoignant de la qualité de la reconstruction.

Les mélanocytes sont des mélanocytes provenant de peau humaine adulte, jeune ou de nouveau-né, en particulier de peau humaine adulte. De tels mélanocytes expriment l'enzyme TRP-2, contrairement aux mélanocytes du follicule pileux. La nature de la mélanine synthétisée est donc différente : les mélanocytes dans l'épiderme produisent de l'eumélanine composée de DHI-mélanine et de DHICA-mélanine alors que les mélanocytes dans le cheveu produisent principalement que de la DHI-mélanine du fait de l'absence de l'expression de TRP-2.
Par ailleurs, le principal stimulus physiologique des mélanocytes de la peau est le rayonnement UV, qui en activant la mélanogénèse et la redistribution de la mélanine, induit un brunissement de la peau, le bronzage. Cette fonctionnalité physiologique ne concerne pas les mélanocytes du cheveu dont la pigmentation n'est pas induite par les UVs.
Les mélanocytes peuvent provenir de peau caucasienne, asiatique ou africaine, de différents sites anatomiques (tels que notamment dos, visage , sein, dos des mains, paumes etc..), de zones ayant été exposées ou non au soleil. On peut utiliser des mélanocytes provenant de peaux normales. Selon un mode particulier de mise en oeuvre de l'invention, on peut aussi utiliser des mélanocytes provenant de pathologies cutanées telles que par exemple taches de vieillesse (lentigo actinique), melasma, vitiligo, nevus, xeroderma pigmentosum ou mélanome. Ils peuvent être aussi des mélanocytes génétiquement modifiés surexprimant ou sous-exprimant certains gènes.

Les mélanocytes localisés dans la couche basale produisent des mélanosomes et de la mélanine qui sont transférés dans les kératinocytes voisins. On peut ainsi reproduire le phénotype des peaux présentant une pigmentation constitutive plus ou moins intense liée au type de mélanocytes. La pigmentation constitutive peut être quantifiée par toute méthode de mesure de la pigmentation ou de la quantité de mélanine, comme par exemple, par spectrocolorimétrie (mesure de la luminance), par mesure en chromatographie liquide haute performance HPLC, par spectrophotométrie visible après dissolution de la peau par le Soluène ou la soude ou par analyse d'image après coloration de la mélanine par Fontana-Masson, par imagerie confocale ou mutliphotonique.
Avantageusement, l'équivalent de peau selon l'invention présente une luminance, mesurée selon la technique de Chardon et al. (In Biological responses to ultraviolet A radiation, Ed Urbach 1992) inférieure ou égale à 80, en l'absence de toute exposition aux UV et/ou à un agent propigmentant. Cette valeur pourra être plus faible, en particulier dans le cas d'équivalent de peau africaine, et pourra notamment varier de +80 à -40 pour des phénotypes allant de très clair à noir. En général, les peaux reconstruites pigmentées selon l'invention présentent un écart de luminance (Delta L) d'au moins 1,5 par rapport à une peau reconstruite ne comprenant pas de mélanocytes, et ce en l'absence de toute irradiation U.V.

L'équivalent de derme selon l'invention comprend des fibroblastes dont les axes principaux sont orientés dans au moins deux directions perpendiculaires. Avantageusement, le pourcentage des fibroblastes orientés longitudinalement par rapport à la surface de l'équivalent de derme est inférieur à 50%.

L'équivalent de derme comprend une matrice ou lattice libre de collagène, contractile dans toutes les directions, homogène sans biopsie; les fibroblastes - et le cas échéant d'autres cellules du derme- sont répartis dans un gel continu de collagène.
L'équivalent de derme comporte au moins une matrice de collagène de type I dans laquelle sont répartis les fibroblastes. Elle peut contenir en outre d'autres constituants de la matrice extracellulaire. Par 'constituant de matrices extracellulaires', on désigne notamment des molécules telles que les collagènes en particulier le collagène IV, les laminines, l'entactine, la fibronectine, les protéoglycanes, les glycosaminoglycans, l'acide hyaluronique. Selon l'un des modes de réalisation de l'invention, l'équivalent de derme contient au moins du collagène IV et de la laminine; de préférence, il contient aussi de l'entactine. Les concentrations de ces différents constituants pourront être adaptées par l'homme du métier et seront par exemple pour la laminine comprises entre 1 et 15% du volume final), pour le collagène IV entre 0.3 et 4.5% du volume final et pour l'entactine entre 0.05 et 1% du volume final.
Le collagène utilisé peut être du collagène d'origine bovine, de queue de rat ou de poisson ou toute autre source de collagène natif ou produit par génie génétique permettant une contraction en présence de fibroblastes.
La matrice est un gel de collagène non tendu, obtenue par contraction aussi bien horizontalement que verticalement, n'imposant pas d'organisation préférentielle des fibroblastes. Une telle matrice dite aussi "libre" n'adhère pas au support et ses volumes peuvent être modifiés à volonté, lui conférant une épaisseur et un diamètre variable; l'épaisseur de l'équivalent de derme sera généralement d'au moins 0,05 cm et notamment d'environ de 0.05 à 2 cm, mais pourra être augmentée sans nuire aux propriétés avantageuses de l'équivalent de peau selon l'invention ; de même, son diamètre sera adapté par l'homme du métier d'environ 3 mm à 20 cm ou plus.

Entre le compartiment dermique comprenant les fibroblastes vivants et l'équivalent d'épiderme multistratifié, le contact est direct et constitue une jonction dermo-épidermique proche de celle existant in vivo tant sur le plan structural que biochimique. Sur le plan biochimique, elle comprend des composants de la membrane basale ; de la lamina densa, de la lamina lucida et de la zone sub-basale tels que, entre autres, le collagène IV, le collagène VII, la laminine 5, l'entactine, la fibronectine.

L'équivalent de peau, utile dans le procédé d'évaluation selon l'invention, peut aussi contenir des cellules endothéliales, des cellules de papilles dermiques, des cellules du système immunitaire, telles que des lymphocytes, des cellules dendritiques, des macrophages ou des cellules de Langerhans, des adipocytes, des cellules nerveuses, et leurs mélanges.

Comme indiqué plus haut, l'équivalent de peau présente une pigmentation constitutive, et la pigmentation peut en outre être augmentée par exposition à un agent pro-pigmentant et/ou à des rayonnements U.V.
En particulier, après stimulation par les UV à des doses équivalentes à l'irradiation quotidienne ou zénithale reçues par une peau humaine d'un individu en conditions physiologiques, les fibroblastes du modèle selon l'invention produisent des facteurs cytosolubles tels que des cytokines ou des facteurs de croissance qui participent à la pigmentation et/ou à sa modulation.

De plus, le nombre de mélanocytes dans la couche basale augmente après stimulation par les UV, notamment d'un facteur d'au moins 1.5, et leur morphologie est modifiée : ils présentent une augmentation de la dendricité marquée par une augmentation du nombre de dendrites ainsi que leur allongement ; la quantité de mélanine est également augmentée par synthèse et par augmentation du transfert dans les couches épidermiques d'un facteur supérieur ou égal à 1,5.
L'augmentation de la pigmentation est évaluée par mesure de la variation de luminance de la peau ou toute autres méthodes de quantification de la mélanine comme par exemple, par mesure en chromatographie liquide haute performance HPLC, par spectrophotométrie visible après dissolution de la peau par le Soluène ou la soude, par analyse d'image après coloration de la mélanine par Fontana-Masson ou par imagerie confocale ou multiphotonique.

La fonctionnalité physiologique du modèle selon l'invention après UV, est donc montrée par les changements des paramètres suivants :
- la couleur macroscopique : les peaux exposées aux UV sont nettement plus foncées que les peaux non exposées : cette stimulation macroscopique de la pigmentation est quantifiée par la diminution de la Luminance (DL = 4. 38).
- la densité des mélanocytes: la densité des mélanocytes après UV est augmentée comme dans la peau normale.
- la quantité de mélanine: la pigmentation est réellement stimulée après UV : la quantité de mélanine est augmentée et cette augmentation est quantifiée par analyse d'images, après coloration Fontana-Masson (coloration de révélation des grains de mélanine) ou par dosage fe la mélanine après extraction au soluène ou à la soude.

L'invention a également pour objet un procédé de préparation d'un tel équivalent de peau. Le procédé comprend notamment les étapes suivantes :
a) mise en contact de fibroblastes et d'une solution de collagène, puis incubation pendant un temps suffisant pour obtenir une matrice de collagène contracté dans laquelle sont répartis les fibroblastes, constituant un équivalent de derme,
b) co-ensemencement par un mélange de kératinocytes et de mélanocytes, de l'équivalent de derme obtenu en a), et culture en immersion dans un milieu liquide,
c) émersion de l'ensemble de la culture (kératinocytes et mélanocytes ensemencés sur l'équivalent de derme) obtenue en b), et poursuite de la culture à l'interface air-liquide jusqu'à obtention d'un équivalent d'épiderme pluristratifié contenant des mélanocytes, sur un équivalent de derme contenant des fibroblastes dans une matrice de collagène, constituant un équivalent de peau.
De préférence l'équivalent d'épiderme pluristratifié forme des couches cornées et les mélanocytes sont localisés sur l'assise basale de l'épiderme.

L'équivalent de peau ainsi obtenue peut être prélevé ou utilisé sur son support dans différents procédés d'évaluation.

L'étape a peut être effectuée avec du collagène de type I, notamment d'origine bovine, ou un mélange de collagènes I et III, (30 % environ par rapport au volume final de la lattice) en suspension homogène. Avantageusement, on y ajoute d'autres constituants tels que laminine (notamment de 1 à 15 % par rapport au volume final), collagène IV (notamment de 0.3% à 4.5% par rapport au volume final) et/ou entactine (notamment de 0.05% à 1% par rapport au volume final) pour obtenir une suspension homogène.
Les fibroblastes sont obtenus à partir de peau humaine, avantageusement de peau adulte. Il peut s'agir de fibroblastes papillaires et/ou réticulaires, seuls ou en mélange en toute proportion, issus de peau caucasienne, asiatique ou africaine, de différents sites anatomiques ( dos, visage, sein, dos des mains, paumes etc..), de zones ayant été exposées ou non au soleil. Dans un mode de réalisation particulier, on utilise des fibroblastes provenant de pathologies cutanées telles que par exemple taches de vieillesse (lentigo actinique), melasma, vitiligo, nevus, mélanome ou xeroderma pigmentosum. Ils peuvent être aussi des fibroblastes génétiquement modifiés surexprimant ou sous-exprimant certains gènes.

Ils sont cultivés dans un milieu adapté, connu de l'homme du métier, puis mis en suspension avant mélange avec la suspension de collagène et des facteurs de croissance. Le mélange est incubé pendant 1 à 6 jours, de préférence pendant 4 ou 5 jours, à une température d'environ 37°C, généralement comprise entre 36°C et 37,5°C. Avantageusement, le mélange est incubé sur un support ne permettant pas son adhésion, en particulier empêchant l'adhésion du mélange aux bords du support; un tel support peut notamment être obtenu par un traitement préalable de sa surface, par exemple par revêtement dudit support par du sérum ou de l'albumine bovine. On obtient ainsi un gel de collagène qui s'est contracté librement dans plusieurs directions, en expulsant le milieu nutritif, et dans lequel sont enchâssés les fibroblastes.

Pour réaliser l'étape b, on utilise des kératinocytes provenant de peau humaine, de préférence de peau adulte humaine. Ils peuvent provenir de peau caucasienne, asiatique ou africaine, de différents sites anatomiques (dos, visage, sein, dos des mains, paumes etc..), de zones ayant été exposées ou non au soleil. Dans un mode de réalisation particulier, on utilise des kératinocytes provenant de pathologies cutanées telles que par exemple taches de vieillesse (lentigo actinique), melasma, vitiligo, nevus ou mélanome. Ils peuvent être aussi des kératinocytes génétiquement modifiés surexprimant ou sous-exprimant certains gènes.

Une préparation de kératinocytes peut être obtenue selon les méthodes classiques de culture cellulaire.
En particulier, on pourra, à partir d'un explant cutané prélevé sur un sujet, procéder comme suit :
- on élimine le tissu sous-cutané à l'aide d'un scalpel ;
- on décontamine le prélèvement cutané par un traitement antibiotique (ex : gentamycine) ;
- on sépare le derme de l'épiderme par traitement protéolytique (ex : trypsine et dispase) puis dissection ;
- on favorise ensuite la dissociation des cellules en présence d'une solution de trypsine 0,05% et EDTA 0,02% ; et on neutralise l'effet de la trypsine par l'ajout d'un milieu de culture DMEM contenant 10% de sérum ;
- on homogénéise la suspension cellulaire, que l'on lave ensuite dans du milieu de culture pour kératinocytes selon la technique de Rheinwald et Green (Cell, 1975).

Les kératinocytes sont amplifiés avant ensemencement selon la technique de Rheinwald et Green (Cell, vol 6 , 331-344,1975) par culture sur un support nourricier constitué de fibroblastes 3T3 dans un milieu adapté connu de l'homme du métier, en présence de facteurs de croissance, notamment d'acides aminés, sérum, toxine cholérique, insuline, tri-iodo-thyronine et solution tampon de pH. En particulier, un tel milieu de culture pourra notamment contenir au moins un facteur de croissance mitogénique pour les kératinocytes (par exemple epidermal growth factor (EGF) et/ou keratinocyte growth factor (KGF), en particulier du KGF), de l'insuline, de l'hydrocortisone et facultativement un antibiotique (ex : gentamycine, amphotericine B).
Avantageusement, ledit milieu pourra comprendre en outre du sérum ou un extrait pituitaire, par exemple d'origine bovine, de l'épinephrine, de la transferrine et/ou des acides aminés non essentiels.

Les mélanocytes sont des mélanocytes provenant de peau humaine, jeune ou adulte, issus de peau caucasienne, asiatique ou africaine, de différents sites anatomiques (dos, visage, sein, prépuce, dos des mains, paumes etc..), de zones ayant été exposées ou non au soleil. Dans un mode de réalisation particulier, on utilise des mélanocytes provenant de pathologies cutanées telles que par exemple taches de vieillesse (lentigo actinique), melasma, vitiligo, nevus, mélanome. Ils peuvent être aussi des mélanocytes génétiquement modifiés surexprimant ou sous-exprimant certains gènes.

Ils sont amplifiés par culture dans un milieu adapté en l'absence d'ester de phorbol composé d'un milieu de base tels que le DMEM/F12 ou le MCDB153 et additionné de facteurs de croissance spécifiques aux mélanocytes (tels que par exemple bFGF, SCF, ET-1, ET3, αMSH) et en particulier dans le milieu M2 (Promocell) ou dans d'autres milieux tel que le M254 (Cascades Biologics ™).
Des suspensions cellulaires de mélanocytes et de kératinocytes sont préparées à partir de ces cultures, et mélangées pour obtenir des suspensions mixtes kératinocytes/mélanocytes. Le ratio mélanocytes/kératinocytes peut être compris entre 1:10 et 2:1, et est généralement d'environ 1:1.

Le procédé de préparation d'un équivalent de peau selon l'invention comporte donc des étapes au cours desquelles les kératinocytes et les mélanocytes sont amplifiés séparément dans leur propre milieu de croissance. Les cellules sont ensuite détachées et co-ensemencées directement sur l'équivalent dermique à densité et ratio définis.
Cette méthode implique une plus grande flexibilité et un contrôle possible de chacun des types cellulaires pendant l'amplification et pendant l'ensemencement des cellules épidermiques sur le derme équivalent. Ce contrôle de la quantité de chacun des types cellulaires ensemencés ainsi que des ratios représente un avantage pour l'application industrielle en terme de contrôle de la reconstruction épidermique et de reproductibilité.

On voit donc que selon des variantes de l'invention, on peut utiliser des kératinocytes et des mélanocytes de peau normale, ou bien utiliser des mélanocytes et des kératinocytes provenant de pathologies cutanées, voire l'un des 2 types cellulaires provenant de pathologies cutanées et l'autre correspondant à des cellules normales, saines.

Cette suspension mixte est déposée sur l'équivalent de derme; l'équivalent de derme est avantageusement fixé sur un support par un matériau biologique tel que du collagène. La suspension mélanocytes/kératinocytes est déposée dans un anneau ou tout moyen équivalent permettant son maintien sur une partie de surface délimitée. Un milieu nutritif liquide est ajouté de façon à recouvrir le mélange de cellules; ce milieu contient des facteurs de croissance connus de l'homme du métier, en particulier EGF et/ou KGF. Le milieu sera renouvelé régulièrement et la culture poursuivie en immersion, généralement pendant une durée comprise entre 2 et 10 jours, notamment de 5 à 8 jours, et d'environ 7 jours. Le milieu contiendra avantageusement du KGF à partir du 2^{ème} jour d'immersion et idéalement à partir du 4^{ème} jour d'immersion.

Comme indiqué précédemment, l'équivalent de derme pourra comprendre des fibroblastes normaux et/ou d'origine pathologique. Ces différents types de derme pourront être associés à un équivalent d'épiderme préparé à partir de mélanges kératinocytes/mélanocytes normaux ou pathologiques, selon les diverses variantes décrites plus haut.
Les peaux sont ensuite, de manière connue en soi, mises en émersion pour obtenir la différenciation des kératinocytes et la formation d'un équivalent d'épiderme stratifié. Cette étape c correspondant à la culture en émersion à l'interface air-liquide est poursuivie jusqu'à obtention d'une structure différenciée, en général environ 7 jours. Toutefois, l'étape c peut être poursuivie plus longtemps, par exemple pendant environ 28 jours tout en conservant un équivalent de peau présentant les caractéristiques avantageuses précisées dans ce qui précède. Le milieu de culture nutritif sera renouvelé régulièrement. L'équivalent de peau est ensuite prélevé pour effectuer des tests requis.
Le procédé de préparation ne met pas en oeuvre de substance mutagène telle que le TPA ou PMA, et les cellules ne présentent pas de modifications de type tumoral.
Le modèle de peau susceptible d'être obtenu selon l'invention présente une pigmentation constitutive, et une fonctionnalité permettant une pigmentaion inductible, et ce pour un modèle in vitro, en l'absence de toute greffe sur un organisme vivant plus complet.

Le modèle de peau selon l'invention permet d'appréhender la voie de régulation de la mélanogénèse dans sa globalité. Ceci peut se traduire par une activité directe sur le mélanocyte mais aussi par une activité (pro ou anti pigmentante) médiée par le kératinocyte, le fibroblaste, l'ensemble du contexte tri-dimensionnel et l'influence du micro-environnement.

Il est utile en particulier dans différents procédés d'évaluation.
L'agent à évaluer dans la mise en oeuvre des procédés selon l'invention pourra être toute substance ou composé chimique ou biologique, ou tout traitement ou phénomène physique susceptible ou apte à moduler la pigmentation, directement ou indirectement. A titre d'exemple de traitement physique susceptible d'être évalué selon l'invention, on peut mentionner un rayonnement lumineux, par exemple UV ou IR, notamment avec un traitement LED (light emitting diode).
Par application de l'agent à l'équivalent de peau on entend le fait de provoquer une interaction entre l'agent à évaluer et au moins une partie de l'équivalent de peau, que ce soit par contact direct ou par tout autre moyen.

L'invention se rapporte en particulier à une méthode de criblage d'un agent susceptible de moduler la pigmentation, ledit agent pouvant être un composé ou un mélange de composés, obtenu notamment par synthèse chimique ou par extraction à partir de matière première d'origine végétale ou bactérienne/levure. Le procédé sera particulièrement utile pour cribler et identifier des actifs utiles dans le domaine de la cosmétique. Les agents peuvent ainsi être toutes molécules, facteurs de croissance, dérivés rétinoïques, hormones, extraits végétaux, pouvant intervenir sur le compartiment épidermique , dermique ou jonctionnel. Les modulateurs peuvent être des siRNA ou des antagomirs.

Le procédé pourra également être utile pour évaluer la modulation de la pigmentation d'actifs utiles dans le domaine pharmaceutique, notamment en dermatologie.

Selon un autre aspect, le procédé selon l'invention permettra d'évaluer les modifications des différents facteurs impliqués lors de l'application d'un agent modulant la pigmentation.

Par moduler la pigmentation on entend selon l'invention modifier le niveau de pigmentation constitutive ou induite de la peau, soit en l'augmentant, soit en la diminuant ou en l'inhibant, que ce soit directement ou indirectement.
Dans le procédé selon l'invention, l'équivalent de peau témoin sera préparé et conservé dans des conditions identiques à celles de l'équivalent de peau auquel on applique l'agent à évaluer, et la mesure de la pigmentation sera effectuée à des temps identiques sur les équivalents de peau témoin et celui ayant reçu l'agent à évaluer.

Selon un mode de réalisation, l'agent à évaluer est appliqué par contact direct avec au moins une partie de l'équivalent de peau. Selon un autre mode de réalisation, l'agent peut également être appliqué dans le milieu de culture dudit équivalent de peau, voire avec un moyen qui provoquera une interaction indirecte de l'agent avec l'un des constituants de l'équivalent de peau. A titre non limitatif de mode d'application alternatifs de l'agent à l'équivalent de peau, on peut citer notamment l'injection, la mésothérapie ou l'iontophorèse.
L'agent à évaluer peut en particulier être appliqué sur un équivalent de peau comportant au moins des fibroblastes et/ou des kératinocytes et/ou des mélanocytes provenant de désordres pigmentaires tels que les taches de vieillesse (lentigo actinique).

Selon l'un des modes de mise en oeuvre du procédé, l'agent à évaluer est un agent inhibant la pigmentation, dont on veut mesurer l'activité dépigmentante et/ou anti-pigmentante. Le procédé permet notamment d'évaluer l'effet de produits destinés à prévenir, ralentir ou réduire les tâches pigmentaires et/ou d'autres désordres pigmentaires.
Le procédé permet selon l'un de ses aspects d'évaluer un agent dépigmentant, c'est-à dire d'évaluer la capacité dudit agent à diminuer la pigmentation constitutive ou induite. Selon un autre aspect, le procédé vise à évaluer un agent anti-pigmentant, c'est-à-dire de tester la capacité dudit agent à prévenir et/ou réduire la pigmentation induite.

Selon un aspect de l'invention, le produit à évaluer sera appliqué sur un modèle pathologique tel que décrit dans ce qui précède, par exemple de désordres hyper ou hypopigmentés tels que le vitiligo, le mélanome, le nevus, le lentigo actinique, le mélasma .... à partir de cellules (mélanocytes mais aussi kératinocytes ou fibroblastes) isolées de ces pathologies ou modifiées génétiquement (silencing, sur expression,...).

Selon un autre mode de réalisation du procédé selon l'invention, l'agent à évaluer est un agent favorisant la pigmentation, seul ou en combinaison avec le rayonnement U.V.

Selon un mode de réalisation particulier, les équivalents de peau sont soumis en outre à une exposition à des rayonnements U.V., les rayonnements U.V. étant appliqués avant, concomitamment et/ou après l'application de l'agent à évaluer, ou pendant une période de temps équivalente sur l'équivalent de peau témoin.
Le procédé selon l'invention sera notamment utile pour évaluer des filtres solaires.
L'invention permettra aussi d'évaluer le pouvoir photoprotecteur d'un agent modulant la pigmentation. On comparera alors le niveau de dommages induits par l'exposition UV avant ou après stimulation ou inhibition de la pigmentation par l'agent., notamment sur la formation des sunburn cells, des dommages à l'ADN, du stress oxydatif, etc...

Comme indiqué plus haut, l'équivalent de peau présente une pigmentation constitutive, et la pigmentation peut en outre être augmentée par exposition à un agent pro-pigmentant et/ou à des rayonnements U.V.
En particulier, après stimulation par les UV à des doses équivalentes à l'irradiation quotidienne ou zénithale reçues par une peau humaine d'un individu en conditions physiologiques, les fibroblastes du modèle selon l'invention produisent des facteurs cytosolubles tels que des cytokines ou des facteurs de croissance qui participent à la pigmentation et/ou à sa modulation.

De plus, le nombre de mélanocytes dans la couche basale augmente après stimulation par les UV, notamment d'un facteur d'au moins 1.5, et leur morphologie est modifiée : ils présentent une augmentation de la dendricité marquée par une augmentation du nombre de dendrites ainsi que leur allongement ; la quantité de mélanine est également augmentée par synthèse et par augmentation du transfert dans les couches épidermiques d'un facteur supérieur ou égal à 1,5.
L'augmentation de la pigmentation est évaluée par mesure de la variation de luminance de la peau ou toute autres méthodes de quantification de la mélanine comme par exemple, par mesure en chromatographie liquide haute performance HPLC, par spectrophotométrie visible après dissolution de la peau par le Soluène ou la soude, par analyse d'image après coloration de la mélanine par Fontana-Masson ou par imagerie confocale ou multiphotonique.

Le procédé selon l'invention permet d'évaluer l'effet des UV sur la pigmentation qui peuvent agir directement sur les mélanocytes et/ou indirectement sur les kératinocytes qui vont à leur tour activer les mélanocytes (action sur la mélanogénèse, la prolifération ou l'adhésion des mélanocytes)
Il permet en plus de manière unique d'évaluer l'impact sur la pigmentation des UV qui peuvent agir :
- sur les fibroblastes, les protéines de la matrice extracellulaire, et les éléments de la jonction dermo-épidermique. En réponse, ces facteurs ou modifications induits peuvent stimuler directement les mélanocytes et/ou indirectement les kératinocytes qui à leur tour vont stimuler les mélanocytes
- sur les kératinocytes qui à leur tour vont moduler les fibroblastes, les protéines de la matrice extracellulaire, et les éléments de la jonction dermo-épidermique et en conséquence la pigmentation.

Il permet également de tester des produits ou des molécules qui interfèrent avec la pigmentation constitutive ou induite par le soleil (bronzage) : freiner, inhiber ou stimuler, par voie topique et systémique
- dépigmentants, antipigmentants
- propigmentants (activateurs de la pigmentation seuls ou avec les UV)

Selon une variante du procédé d'évaluation selon l'invention, on réalise en outre un groupe de mesure supplémentaire avec des produits de référence connus pour leur activité favorisant ou au contraire inhibant la pigmentation.

Dans cette variante, on compare en outre la pigmentation (i) de l'équivalent de peau *in vitro* auquel a été appliqué l'agent à évaluer, à celle (iii) d'un équivalent de peau auquel a été appliqué une substance de référence favorisant ou inhibant la pigmentation.
On pourra par exemple choisir de retenir les agents pour lesquels la pigmentation est au moins supérieure ou égale à celle observée pour la substance de référence, dans le cas d'un agent favorisant la pigmentation. Inversement, on pourra retenir les agents pour lesquels la pigmentation est inférieure ou égale à celle obtenue avec la substance de référence dans le cas d'un agent dépigmentant.
On pourra également comparer la variation de pigmentation mesurée d'une part entre l'équivalent de peau auquel a été appliqué l'agent à évaluer et l'équivalent de peau témoin (i)-(ii) et d'autre part l'équivalent de peau auquel a été appliqué la substance de référence et l'équivalent de peau témoin (iii)-(ii); les agents sélectionnés seront ceux pour lesquels la modulation de la pigmentation sera au moins égale à celle mesurée pour la substance de référence.

Selon un mode de mise en oeuvre particulier, l'équivalent de peau *in vitro* comprend au moins des kératinocytes, mélanocytes et/ou des fibroblastes modifiés génétiquement, notamment par surexpression de cDNA, par extinction de gène par mutagénèse dirigée, ou par dsRNA,selon la technique des siRNA ou expression par shRNA.

Selon un autre mode de réalisation, l'équivalent de derme comprend un équivalent de matrice extracellulaire, les constituants dudit équivalent de matrice extracellulaire comprenant au moins du collagène de type I, et les constituants ont subi au moins une modification par rapport à un équivalent de matrice extracellulaire de peau jeune, saine. Il peut s'agir par exemple de modification de la nature, de la quantité ou des proportions des macromolécules constituant de l'équivalent de derme, et/ou de modifications chimiques. On pourra ainsi faire varier la composition de l'équivalent de derme, et/ou les proportions notamment de collagène IV, laminines, entactine, fibronectine, protéoglycanes, glycosaminoglycans et/ou acide hyaluronique présents, au-delà des concentrations classiquement utilisées et indiquées dans ce qui précède. Les constituants de l'équivalent de matrice extracellulaire peuvent en particulier avoir subi une modification chimique choisie parmi la glycation, la réticulation et l'oxydation

Le procédé selon l'invention permet d'appréhender la voie de régulation de la mélanogénèse dans sa globalité. Ceci peut se traduire par une activité directe sur le mélanocyte mais aussi par une activité (pro ou anti pigmentante) médiée par le kératinocyte, le fibroblaste, l'ensemble du contexte tri-dimensionnel et l'influence du micro-environnement.

Il permet d'évaluer l'impact sur la pigmentation d'agents qui peuvent agir :
- sur les fibroblastes, les protéines de la matrice extracellulaire, et les éléments de la jonction dermo-épidermique. En réponse, ces facteurs ou modifications induits peuvent stimuler directement les mélanocytes et/ou indirectement les kératinocytes qui à leur tour vont stimuler les mélanocytes
- sur les kératinocytes qui à leur tour vont moduler les fibroblastes, les protéines de la matrice extracellulaire, et les éléments de la jonction dermo-épidermique et en conséquence la pigmentation.
Le procédé peut ainsi être utile pour
- Tester l'impact sur la pigmentation de cellules issues de désordres hyper ou hypopigmentés tels que le vitiligo, le mélanome, le nevus, le lentigo actinique, le mélasma .... à partir de cellules (mélanocytes mais aussi kératinocytes ou fibroblastes) isolées de ces pathologies ou modifiées génétiquement (silencing, sur expression,...)
- étudier l'influence de dérégulations kératinocytaires ou fibroblastiques (kératinocytes ou fibroblastes pathologiques) sur la pigmentation.
- pour étudier l'influence de dérégulations de la matrice extracellulaire sur la pigmentation (glycation, pontages (cross-link), oxydation des macromolécules de la matrice extracellulaire, ou modifications de la nature, de la quantité et de la proportion des macromolécules.

L'invention concerne également un kit de production d'une peau reconstruite comprenant un support de derme, des mélanocytes et des kératinocytes, ainsi que les milieux décrits plus hauts.
L'invention va être illustrée plus en détail dans les exemples qui suivent. Dans ces exemples, on se référera aux figures suivantes :
Figure 1 : Peau reconstruite pigmentée humaine
Figure 2 : Peau reconstruite pigmentée humaine avec une pigmentation constitutive liée au type de mélanocyte
Figure 3 : Induction de la pigmentation par les UV dans la peau reconstruite pigmentée
Figure 4 : Induction de la pigmentation par l'alpha MSH dans la peau reconstruite pigmentée

### Exemple 1: préparation des équivalents de peau

### Préparation des lattices (J-4)

Les dermes équivalents sont réalisés avec du collagène I bovin et des fibroblastes humains issus de derme.

Les dermes équivalents sont réalisés avec du collagène I bovin et des fibroblastes humains issus de derme selon les techniques décrites par Asselineau et coll 1985 (Exp Cell Res. vol 159, 536-539) et Bernerd et Asselineau 1997 (Dev Biol, vol 183, 123-138). Un coating de la boite de Petri est préalablement réalisé avec du MEM contenant 10% de serum.
Avant de couler la préparation de collagène contenant les cellules, une solution de laminine (3% /volume final) de collagène IV (1,5 %/ volume final) et d'entactine (0,35% / volume final) est ajoutée au mélange qui est vigoureusement agité. Cette suspension est placée à l'étuve (37°C - 5 % CO2) pour permettre à la lattice de se contracter.

### Ensemencement des kératinocytes et des mélanocytes (J0)

L'ensemencement en kératinocytes et mélanocytes sur le derme équivalent se fait après contraction des lattices.

Pour cela, les cellules sont amplifiées 7 jours avant ensemencement, dans leur milieu de croissance respectif:
Les mélanocytes sont amplifiés dans un milieu de culture pour mélanocytes, le Milieu M2 (Promocell) en l'absence de tout ester de phorbol.

Les kératinocytes sont amplifiés selon la technique de Rheinwald et Green, Cell, vol 6, 331-344,1975 par culture sur un support nourricier constitué de fibroblastes 3T3

Pour l'ensemencement des kératinocytes et des mélanocytes sur le derme équivalent, la lattice est d'abord collée au fond d'une boite à l'aide d'une préparation à base de collagène (pour 2 lattices, 0.46 ml MEM 1.76 X+ 0.09 ml FCS + 0.05 ml NaOH 0.1 N+ 0.1 ml MEM Hépes 10% FCS + 0.3 ml de collagène dialysé).

Les mélanocytes et les kératinocytes sont trypsinés et les suspensions cellulaires sont ajustées à une concentration de 200 000 cellules / ml chacune avec du milieu constitué de MEM 10 % FCS + 2 mM L-Glutamine +1 mM Sodium Pyruvate , +1 X Acides aminés non essentiels ,+ 0.2% Penicilline Streptomycine + 0.1% antibiotique anti-mycotique +10 ng/ml EGF , 10⁻¹⁰ M Choléra Toxine + 0.4 µg/ml Hydrocortisone + 0.625µl/ml de SupplementMix (Promocell) = milieu PRP).

Une suspension mixte de mélanocytes et de kératinocytes est préparée avec 0.25ml de la suspension de kératinocytes et 0.25ml de suspension de mélanocytes. La suspension finale contenant donc 50 000 kératinocytes et 50 000 mélanocytes est déposée au sein d'un anneau (14 mm de diamètre) placé sur la lattice collée. Autour de l'anneau sont ajoutés 6ml de milieu PRP.
Les boites sont placées à l'étuve 37°C-5% CO₂. Après 2 heures, les anneaux sont retirés. Le même milieu de culture est renouvelé 2 jours plus tard.

4 jours après ensemencement, l'EGF (10 ng/ml) du milieu PRP est remplacé par du KGF (10ng/ml).

### Culture-Emersion des peaux reconstruites pigmentées (J7)

Après 7 jours de culture en immersion les peaux sont mises en émersion sur une grille (interface air-liquide). Le milieu est changé tous les 2 jours.
Après 7 jours d'émersion, les peaux présentant une morphologie très proche de la peau humaine normale sont prêtes pour être prélevées et analysées ou peuvent être maintenues en culture plus longtemps pour différentes expériences avec un changement de milieu tous les 2 jours.

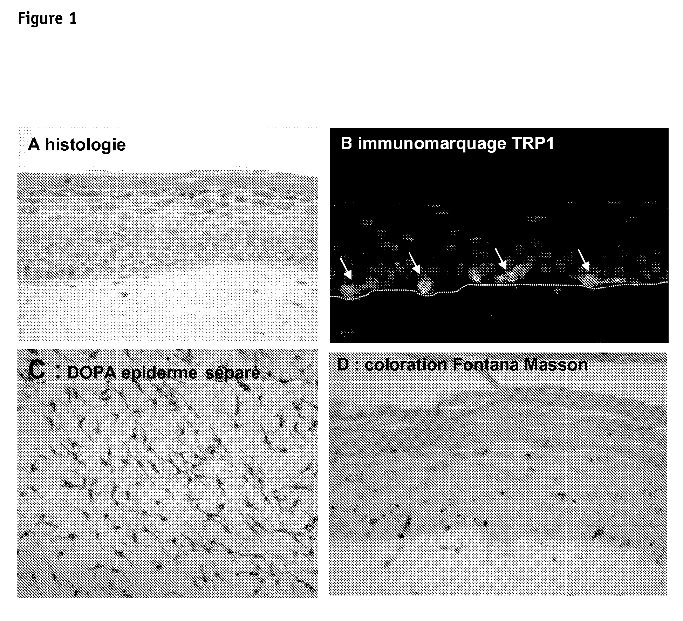

### Exemple 2: Peau reconstruite pigmentée humaine contenant les 3 types cellulaires mélanocytes, kératinocytes et fibroblastes (Figure 1)

La peau après 7 jours d'émersion présente une histologie et une architecture tridimensionnelle proche de celle de la peau humaine.
Elle possède un épiderme stratifié et différencié, constitué des différentes couches cellulaires (basale, épineuse, granuleuse) et une couche cornée.
Le derme comprend des fibroblastes vivants enchassés dans la matrice collagénique (Figure 1A).

Les mélanocytes sont intégrés dans l'épiderme et montrent une morphologie et une densité similaire à celles de la peau normale (figure 1C réaction de DOPA sur épiderme séparé). Ils sont bien localisés dans la couche basale (figure 1B, immunomarquage TRP1, ligne blanche pointillée indiquant la jonction dermo-épidermique), produisent des mélanosomes et de la mélanine qui sont transférés dans les kératinocytes voisins (figure 1D, coloration de la mélanine par Fontana Masson).

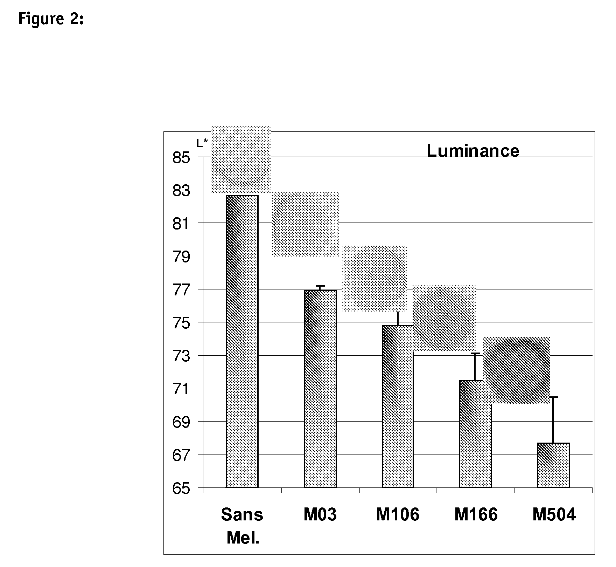

### Exemple 3: Peau reconstruite pigmentée humaine avec une pigmentation constitutive liée au type de mélanocyte (Figure 2)

Le modèle est capable d'intégrer différentes souches de mélanocytes des moins pigmentées aux plus pigmentées (modèle robuste) et le phénotype de la peau reconstruite, plus ou moins pigmenté, correspond au type de mélanocyte utilisé (maintien de la physiologie) : en effet, plus la souche de mélanocyte est pigmentée (de M03 à M504), plus la couleur macroscopique des peaux et la quantité de mélanine visible sur coupe sont intenses. Cette pigmentation macroscopique se traduit par une diminution de la luminance (Figure 2 Luminance).

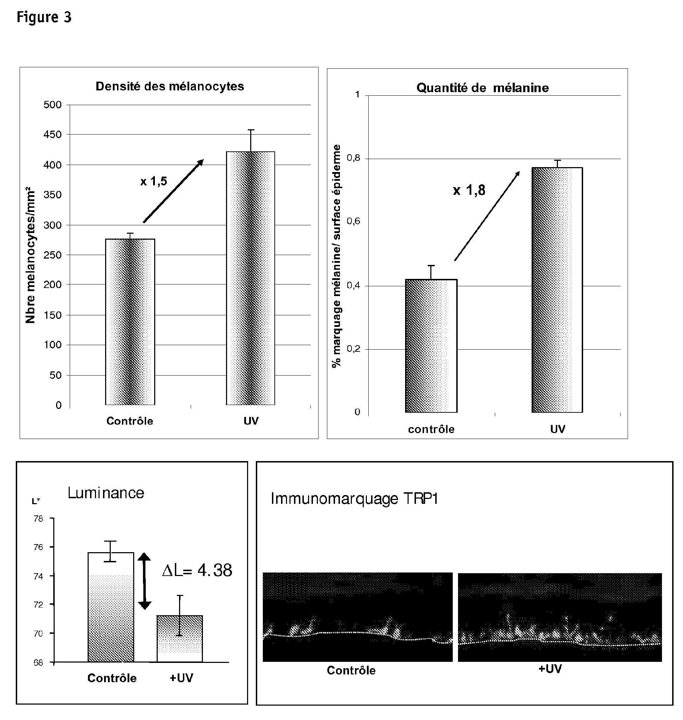

### Exemple 4: Induction de la pigmentation par les UV dans la Peau Reconstruite Pigmentée (Figure 3)

A partir de 7 jours d'émersion, les peaux reconstruites sont soumises aux UVs solaires pour induire la pigmentation. Un simulateur solaire est utilisé : il délivre des UVB et des UVA avec un spectre comparable à celui du soleil (rapport UVB/UVA de 14). Les peaux sont exposées aux UVs 3 fois, une fois tous les 2 jours. Les peaux sont prélevées 48 h après la dernière exposition. Des conditions contrôles sont réalisées en absence d'exposition au simulateur solaire.
Dans les peaux soumises aux UV, les mélanocytes sont toujours présents et bien localisés dans la couche basale (figure 3 immunomarquage de TRP1. la ligne blanche pointillée indique la jonction dermo-épidermique). Ils sont activés par les UV: plus nombreux (figure 3 graphe densité de mélanocytes), plus dendritiques et plus réactifs à la réaction Dopa que dans les peaux non exposées. Une augmentation de la quantité de mélanine est mesurée (x1.8) après coloration Fontana Masson dans les peaux photo-exposées par rapport aux peaux contrôles (figure 3 quantité de mélanine). Cette stimulation de la pigmentation par les UV est quantifiée par la mesure de la luminance(L*) : la diminution de la luminance (ΔL= 4.38) montre que les peaux exposées aux UV sont plus foncées que les peaux non exposées (figure 3 Luminance).

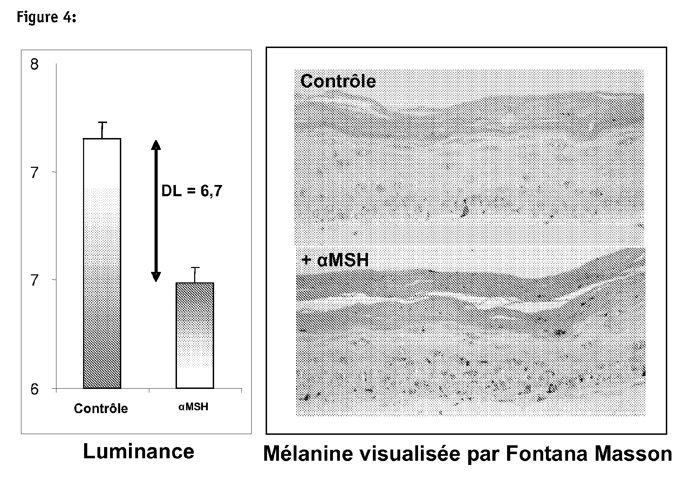

### Exemple 5: Induction de la pigmentation dans la peau reconstruite pigmentée par un agent propigmentant (Figure 4)

En présence d'un agent propigmentant connu, l'αMSH, mis dans le milieu à partir de la phase d'émersion et à la concentration de 50 nM, les peaux prélevées 18 jours après, sont macroscopiquement plus pigmentées. La mesure de la Luminance confirme ce brunissement puisque elle diminue de 6.7 points entre les peaux non traitées et celles traitées par l'αMSH (figure 4 Luminance).
Les mélanocytes sont plus dendritiques et produisent nettement plus de mélanine visible par le marquage Fontana-Masson en présence d'αMSH (figure 4 coloration de Fontana Masson).

## Revendications

1. Equivalent de peau in vitro **caractérisé en ce qu'**il comporte au moins un équivalent d'épiderme qui comprend des kératinocytes formant au moins une couche basale et au moins une couche superficielle et au moins un équivalent de derme comprenant une lattice libre de collagène, et **en ce que** ledit équivalent de peau comprend des mélanocytes produisant de la mélanine de façon constitutive et des fibroblastes vivants, tous les mélanocytes étant dans la couche basale de l'équivalent d'épiderme et distribués de façon homogène dans l'équivalent d'épiderme, la densité de répartition desdits mélanocytes étant sensiblement constante dans un plan parallèle à la surface dudit équivalent.

2. Equivalent de peau in vitro selon la revendication précédente **caractérisé en ce que** dans l'équivalent de derme le pourcentage des fibroblastes orientés longitudinalement par rapport à la surface de l'équivalent de derme est inférieur à 50%.

3. Equivalent de peau in vitro selon l'une au moins des revendications précédentes, **caractérisé en ce que** l'équivalent de derme comporte une matrice de collagène de type I dans laquelle sont répartis les fibroblastes.

4. Equivalent de peau in vitro selon l'une au moins des revendications précédentes, **caractérisé en ce que** les mélanocytes sont des mélanocytes provenant de peau humaine.

5. Equivalent de peau selon l'une quelconque des revendications précédentes, **caractérisé en ce que** sa pigmentation est augmentée par exposition à un agent pro-pigmentant et/ou à des rayonnements U.V.

6. Procédé de préparation d'un équivalent de peau selon l'une des revendications précédentes **caractérisé en ce qu'**il comprend les étapes suivantes:
a) mise en contact de fibroblastes, d'une solution de collagène, puis incubation pendant un temps suffisant pour obtenir une matrice de collagène contracté dans laquelle sont répartis les fibroblastes, constituant un équivalent de derme,
b) co-ensemencement de l'équivalent de derme par un mélange de kératinocytes et de mélanocytes sur l'équivalent de derme obtenu en a), et culture en immersion dans un milieu liquide,
c) émersion de la culture de kératinocytes et de mélanocytes sur l'équivalent de derme obtenu en b), et poursuite de la culture à l'interface air-liquide jusqu'à obtention d'un équivalent d'épiderme pluristratifié contenant des mélanocytes, sur un équivalent de derme contenant des fibroblastes dans une matrice de collagène, constituant un équivalent de peau.

7. Procédé selon la revendication 6, **caractérisé en ce que** les mélanocytes sont des mélanocytes humains, obtenus à partir de peau adulte, et préalablement amplifiés.

8. Procédé selon l'une au moins des revendications 6 ou 7 , **caractérisé en ce que** les kératinocytes et les fibroblastes sont obtenus à partir de peau adulte humaine.

9. Procédé selon l'une au moins des revendications 6 à 8, **caractérisé en ce que** l'étape a) est effectuée en présence de collagène IV, de laminine et/ou entactine.

10. Procédé d'évaluation de la capacité d'un agent à moduler la pigmentation **caractérisé en ce que**
(a) on applique ledit agent à un équivalent de peau *in vitro* selon l'une au moins des revendications 1 à 5,
(b) on compare la pigmentation (i) de l'équivalent de peau *in vitro* auquel a été appliqué l'agent à évaluer à celle (ii) d'un équivalent de peau témoin n'ayant pas été soumis à l'agent.

11. Procédé d'évaluation selon la revendication 10, **caractérisé en ce que** ledit agent est appliqué à l'équivalent de peau par contact direct avec au moins une partie dudit équivalent de peau.

12. Procédé d'évaluation selon l'une au moins des revendications 10 ou 11 **caractérisé en ce que** ledit équivalent de peau comporte au moins des fibroblastes et/ou des kératinocytes et/ou des mélanocytes provenant de désordres pigmentaires.

13. Procédé d'évaluation selon l'une au moins des revendications 10 à 12, **caractérisé en ce que** ledit agent est un agent inhibant la pigmentation.

14. Procédé selon l'une au moins des revendications 10 à 13, **caractérisé en ce que** l'agent à évaluer est appliqué sur des zones de l'équivalent de peau présentant une hyper-pigmentation.

15. Procédé selon l'une au moins des revendications 10 à 12, **caractérisé en ce que** ledit agent à évaluer est un agent favorisant la pigmentation.

16. Procédé d'évaluation selon l'une au moins des revendications 10 à 15, **caractérisé en ce que** les équivalents de peau sont soumis en outre à une exposition à des rayonnements U.V., les rayonnements U.V. étant appliqués avant, concomitamment et/ou après l'application de l'agent à évaluer, ou pendant une période de temps équivalente sur l'équivalent de peau témoin.

17. Procédé d'évaluation selon l'une au moins des revendications 10 à 16, **caractérisé en ce qu'**on compare en outre la pigmentation (i) de l'équivalent de peau *in vitro* auquel a été appliqué l'agent à évaluer, à celle (iii) d'un équivalent de peau auquel a été appliqué une substance de référence favorisant ou inhibant la pigmentation.

18. Procédé d'évaluation selon l'une au moins des revendications 10 à 17, **caractérisé en ce que** l'agent à évaluer module la pigmentation par action au moins en partie sur les kératinocytes, les fibroblastes, les constituants de la matrice extracellulaire et/ou la jonction dermo-épidermique.

19. Procédé d'évaluation selon l'une des revendications 16 à 18, **caractérisé en ce que** l'agent à évaluer comprend au moins un filtre solaire.

## Patentansprüche

1. In vitro-Hautäquivalent,
**dadurch gekennzeichnet, dass** dieses umfasst:
mindestens ein Epidermisäquivalent, das Keratinozyten umfasst, die mindestens eine Basalschicht und mindestens eine Oberflächenschicht bilden, und mindestens ein Dermisäquivalent, das ein freies Kollagengitter umfasst, und dass das Hautäquivalent Melanozyten, die konstitutiv Melanin erzeugen, und lebende Fibroblasten umfasst, wobei sich die gesamten Melanozyten in der Basalschicht des Epidermisäquivalents befinden und homogen in dem Epidermisäquivalent verteilt sind, wobei die Verteilungsdichte der Melanozyten in einer Ebene parallel zu der Oberfläche des Äquivalents im Wesentlichen konstant ist.

2. In vitro-Hautäquivalent nach dem vorhergehenden Anspruch,
**dadurch gekennzeichnet, dass** in dem Dermisäquivalent der Prozentsatz der Fibroblasten, die in Längsrichtung in Bezug auf die Oberfläche des Dermisäquivalents orientiert sind, weniger als 50 % beträgt.

3. In vitro-Hautäquivalent nach mindestens einem der vorhergehenden Ansprüche,
**dadurch gekennzeichnet, dass** das Dermisäquivalent eine Kollagenmatrix Typ I umfasst, in der die Fibroblasten verteilt sind.

4. In vitro-Hautäquivalent nach mindestens einem der vorhergehenden Ansprüche,
**dadurch gekennzeichnet, dass** die Melanozyten Melanozyten sind, die von menschlicher Haut stammen.

5. Hautäquivalent nach einem der vorhergehenden Ansprüche,
**dadurch gekennzeichnet, dass** seine Pigmentierung erhöht wird, indem es einem Pro-Pigmentierungsmittel und/oder UV-Strahlen ausgesetzt wird.

6. Verfahren zur Herstellung eines Hautäquivalents nach einem der vorhergehenden Ansprüche,
**dadurch gekennzeichnet, dass** dieses die folgenden Schritte umfasst:
a) Inberührungbringen von Fibroblasten mit einer Kollagenlösung, anschließend Inkubieren während einer ausreichenden Zeit, um eine kontrahierte Kollagenmatrix zu erhalten, in der die Fibroblasten verteilt sind, wobei ein Dermisäquivalent gebildet wird,
b) Co-Beimpfen des Dermisäquivalents mit einer Mischung von Keratinozyten und Melanozyten auf dem in a) erhaltenen Dermisäquivalent und Kultivieren durch Eintauchen in ein flüssiges Medium,
c) Austauchen der Kultur von Keratinozyten und Melanozyten auf dem in b) erhaltenen Dermisäquivalent und Fortsetzen der Kultur an der Luft-Flüssigkeits-Grenzfläche, bis ein mehrschichtiges Epidermisäquivalent, das Melanozyten enthält, auf einem Dermisäquivalent, das Fibroblasten in einer Kollagenmatrix enthält, erhalten wird, wobei ein Hautäquivalent gebildet wird.

7. Verfahren nach Anspruch 6,
**dadurch gekennzeichnet, dass** die Melanozyten menschliche Melanozyten sind, die aus erwachsener Haut erhalten und vorher amplifiziert werden.

8. Verfahren nach mindestens einem der Ansprüche 6 oder 7,
**dadurch gekennzeichnet, dass** die Keratinozyten und die Fibroblasten aus menschlicher erwachsener Haut erhalten werden.

9. Verfahren nach mindestens einem der Ansprüche 6 bis 8,
**dadurch gekennzeichnet, dass** Schritt a) in Anwesenheit von Kollagen IV, Laminin und/oder Entactin durchgeführt wird.

10. Verfahren zur Evaluierung der Kapazität eines Mittels zur Modulation der Pigmentierung,
**dadurch gekennzeichnet, dass**:
(a) das Mittel bei einem in vitro-Hautäquivalent nach mindestens einem der Ansprüche 1 bis 5 angewendet wird,
(b) die Pigmentierung (i) des in vitro-Hautäquivalents, bei dem das zu evaluierende Mittel angewendet wurde, mit jener (ii) eines Kontroll-Hautäquivalents verglichen wird, das dem Mittel nicht ausgesetzt wurde.

11. Evaluierungsverfahren nach Anspruch 10,
**dadurch gekennzeichnet, dass** das Mittel bei dem Hautäquivalent durch direkten Kontakt mit mindestens einem Teil des Hautäquivalents angewendet wird.

12. Evaluierungsverfahren nach mindestens einem der Ansprüche 10 oder 11,
**dadurch gekennzeichnet, dass** das Hautäquivalent mindestens Fibroblasten und/oder Keratinozyten und/oder Melanozyten umfasst, die von Pigmentstörungen stammen.

13. Evaluierungsverfahren nach mindestens einem der Ansprüche 10 bis 12,
**dadurch gekennzeichnet, dass** das Mittel ein Mittel zur Inhibierung der Pigmentierung ist.

14. Verfahren nach mindestens einem der Ansprüche 10 bis 13,
**dadurch gekennzeichnet, dass** das zu evaluierende Mittel bei den Zonen des Hautäquivalents angewendet wird, die eine Hyperpigmentierung aufweisen.

15. Verfahren nach mindestens einem der Ansprüche 10 bis 12,
**dadurch gekennzeichnet, dass** das zu evaluierende Mittel ein Mittel zur Förderung der Pigmentierung ist.

16. Evaluierungsverfahren nach mindestens einem der Ansprüche 10 bis 15,
**dadurch gekennzeichnet, dass** die Hautäquivalente außerdem einer Belichtung mit UV-Strahlen ausgesetzt werden, wobei die UV-Strahlen vor, gleichzeitig mit und/oder nach der Anwendung des zu evaluierenden Mittels oder während einer äquivalenten Zeitperiode auf das Kontroll-Hautäquivalent angewendet werden.

17. Evaluierungsverfahren nach mindestens einem der Ansprüche 10 bis 16,
**dadurch gekennzeichnet, dass** außerdem die Pigmentierung (i) des in vitro-Hautäquivalents, bei dem das zu evaluierende Mittel angewendet wurde, mit jener (ii) eines Hautäquivalents, bei dem eine Referenzsubstanz angewendet wurde, welche die Pigmentierung fördert oder inhibiert, verglichen wird.

18. Evaluierungsverfahren nach mindestens einem der Ansprüche 10 bis 17,
**dadurch gekennzeichnet, dass** das zu evaluierende Mittel die Pigmentierung durch Einwirken mindestens teilweise auf die Keratinozyten, die Fibroblasten, die Bestandteile der extrazellulären Matrix und/oder die Dermo-Epidermis-Junktion moduliert.

19. Evaluierungsverfahren nach einem der Ansprüche 16 bis 18,
**dadurch gekennzeichnet, dass** das zu evaluierende Mittel mindestens einen Solarfilter umfasst.

## Claims

1. *In vitro* skin equivalent **characterized in that** it comprises at least one epidermis equivalent which comprises keratinocytes forming at least one basal layer and at least one superficial layer, and at least one dermis equivalent comprising a free lattice of collagen, **in that** said skin equivalent comprises melanocytes constitutively producing melanin and live fibroblasts, all the melanocytes being in the basal layer of the epidermis equivalent and distributed homogeneously in the epidermis equivalent, the distribution density of said melanocytes being substantially constant in a plane parallel to the surface of said equivalent.

2. *In* vitro skin equivalent according to the preceding claim, **characterized in that**, in the dermis equivalent, the percentage of fibroblasts oriented longitudinally relative to the surface of the dermis equivalent is less than 50%.

3. *In vitro* skin equivalent according to at least one of the preceding claims, **characterized in that** the dermis equivalent comprises a matrix of collagen type I in which the fibroblasts are distributed.

4. *In vitro* skin equivalent according to at least one of the preceding claims, **characterized in that** the melanocytes are melanocytes originating from human skin.

5. Skin equivalent according to any one of the preceding claims, **characterized in that** the pigmentation thereof is increased by exposure to a propigmenting agent and/or to UV radiation.

6. Method for preparing a skin equivalent according to one of the preceding claims, **characterized in that** it comprises the following steps:
a) bringing fibroblasts, a solution of collagen into contact, then incubating for a sufficient period of time to obtain a contracted collagen matrix in which the fibroblasts are distributed, constituting a dermis equivalent,
b) coseeding the dermis equivalent with a mixture of keratinocytes and melanocytes on the dermis equivalent obtained in a), and immersion culture in a liquid medium,
c) emersion of the culture of keratinocytes and melanocytes on the dermis equivalent obtained in b), and continuation of the culture at the air-liquid interface until a pluristratified epidermis equivalent containing melanocytes, on a dermis equivalent containing fibroblasts in a collagen matrix, constituting a skin equivalent, is obtained.

7. Method according to Claim 6, **characterized in that** the melanocytes are human melanocytes, obtained from adult skin, and pre-amplified.

8. Method according to at least one of Claims 6 or 7, **characterized in that** the keratinocytes and the fibroblasts are obtained from human adult skin.

9. Method according to at least one of Claims 6 to 8, **characterized in that** step a) is carried out in the presence of collagen IV, laminin and/or entactin.

10. Method for evaluating the ability of an agent to modulate pigmentation, **characterized in that**
(a) said agent is applied to an *in vitro* skin equivalent according to at least one of Claims 1 to 5,
(b) the pigmentation (i) of the *in vitro* skin equivalent to which the agent to be evaluated has been applied is compared with that (ii) of a control skin equivalent which has not been subjected to the agent.

11. Evaluation method according to Claim 10, **characterized in that** said agent is applied to the skin equivalent by direct contact with at least one part of said skin equivalent.

12. Evaluation method according to at least one of Claims 10 and 11, **characterized in that** said skin equivalent comprises at least fibroblasts and/or keratinocytes and/or melanocytes originating from pigmentary disorders.

13. Evaluation method according to at least one of Claims 10 to 12, **characterized in that** said agent is a pigmentation-inhibiting agent.

14. Method according to at least one of Claims 10 to 13, **characterized in that** the agent to be evaluated is applied to the areas of the skin equivalent exhibiting hyperpigmentation.

15. Method according to at least one of Claims 10 to 12, **characterized in that** said agent to be evaluated is a pigmentation-promoting agent.

16. Evaluation method according to at least one of Claims 10 to 15, **characterized in that** the skin equivalents are subjected, in addition, to exposure to UV radiation, the UV radiation being applied before, concomitantly with and/or after the application of the agent to be evaluated, or for an equivalent period of time on the control skin equivalent.

17. Evaluation method according to at least one of Claims 10 to 16, **characterized in that** the pigmentation (i) of the *in* vitro skin equivalent to which the agent to be evaluated has been applied is also compared with that (iii) of a skin equivalent to which a reference substance which promotes or inhibits pigmentation has been applied.

18. Evaluation method according to at least one of Claims 10 to 17, **characterized in that** the agent to be evaluated modulates the pigmentation through action at least partly on the keratinocytes, the fibroblasts, the extracellular matrix constituents and/or the dermo-epidermal junction.

19. Evaluation method according to one of Claims 16 to 18, **characterized in that** the agent to be evaluated comprises at least one sunscreen.
